(19) Europäisches Patentamt / European Patent Office / Office européen des brevets

(11) **EP 3 797 700 B1**

(12) **EUROPÄISCHE PATENTSCHRIFT**

(45) Veröffentlichungstag und Bekanntmachung des Hinweises auf die Patenterteilung:
**19.11.2025 Patentblatt 2025/47**

(21) Anmeldenummer: **19200100.6**

(22) Anmeldetag: **27.09.2019**

(51) Internationale Patentklassifikation (IPC):
**A61B 6/04** *(2006.01)*    **A61B 6/00** *(2024.01)*
**A61B 90/50** *(2016.01)*    **B25J 11/00** *(2006.01)*

(52) Gemeinsame Patentklassifikation (CPC):
**A61B 6/4458; A61B 6/0487; A61B 6/548**

(54) **MANIPULATORVORRICHTUNG ZUR AUTOMATISCHEN EINRICHTUNG EINES RADIOLOGIEARBEITSPLATZES**

MANIPULATOR DEVICE FOR AUTOMATICALLY ADJUSTING A RADIOLOGY WORKSTATION

DISPOSITIF MANIPULATEUR DE LA MISE EN PLACE AUTOMATIQUE D'UN POSTE DE TRAVAIL DE RADIOLOGIE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**31.03.2021 Patentblatt 2021/13**

(73) Patentinhaber: **Siemens Healthineers AG**
**91301 Forchheim (DE)**

(72) Erfinder:
• **Hannemann, Thilo**
**91052 Erlangen (DE)**
• **Lerch, Daniel**
**91365 Weilersbach (DE)**
• **Bechtold, Mario**
**91334 Hemhofen (DE)**

(74) Vertreter: **Siemens Healthineers**
**Patent Attorneys**
**Postfach 22 16 34**
**80506 München (DE)**

(56) Entgegenhaltungen:
EP-A1- 3 459 462    EP-A2- 2 302 644
DE-A1- 102010 007 654    DE-A1- 102014 203 465
DE-A1- 102016 225 689    DE-A1- 102017 201 434
JP-A- H0 271 730    US-A1- 2016 249 991

**Beschreibung**

Die Erfindung betrifft eine Manipulatorvorrichtung zur automatischen Einrichtung eines Radiologiearbeitsplatzes, ein medizinisches Bildgebungsgerät und ein Verfahren dazu.

Bei einem vollautomatisierten Radiographiesystem kann die Positionierung der Röntgenröhre, des Detektors, des Tischs und des Rasterwandgeräts durch das Aktivieren einer vorher eingespeicherten Referenzposition, beispielsweise durch die Auswahl eines Organprogramms am Röntgensystem, vollautomatisch erfolgen, falls alle relevanten Achsen des Systems motorisiert ausgebildet sind.

Positionierungshilfen oder mobile Detektoren systemseitig automatisch vorzubereiten, indem diese an die für die jeweilige klinische Prozedur sinnvolle Position (ein-)gelegt werden, ist bisher nicht automatisiert möglich. Das Problem der Kosten mehrerer Detektoren, beispielsweise ein Tischdetektor, ein Rasterwandgerätdetektor und ein frei positionierbarer Detektor, kann bisher so gelöst werden, dass ein Detektor vom Benutzer selbst sowohl in ein Tischeinschubfach oder ein Rasterwandgerät manuell eingesteckt werden kann als auch als frei positionierbarer Detektor genutzt werden kann.

Bei einem radiographischen Arbeitsablauf ist ein wichtiger Schritt die Raumvorbereitung. In diesem Arbeitsschritt bereitet der bzw. die MTRA, also der Benutzer, ausgehend von den angeforderten Aufnahmen und weiteren Informationen über den Patienten den Raum auf die kommende Untersuchung vor.

Aus der Druckschrift DE 10 2016 225689 A1 ist eine Patientenpositioniervorrichtung bekannt, aufweisend eine am Befestigungsflansch des Roboterarms befestigte Kopplungsvorrichtung, die ausgebildet ist, die Patientenliege, durch ein Verschwenken der Kopplungsvorrichtung bezüglich der Patientenliege um eine Schwenkachse während eines Ankoppelns der Kopplungsvorrichtung an die Patientenliege, mit dem Befestigungsflansch des Roboterarms formschlüssig zu verbinden und die Patientenliege, durch ein Verschwenken der Kopplungsvorrichtung bezüglich der Patientenliege in eine entgegen gesetzte Schwenkrichtung um die Schwenkachse während eines Abkoppelns der Kopplungsvorrichtung von der Patientenliege, von dem Befestigungsflansch des Roboterarms zu lösen.

Aus der Druckschrift US 2016/249991 A1 ist ein System und ein Verfahren zur Gewährleistung einer sicheren und erträglichen Einführung einer Nadel in den Körper einer Person gemäß einer vorgeplanten oder kontinuierlich überwachten Abfolge von Einführungsschritten bekannt. Das System umfasst eine Greifvorrichtung zum Ergreifen der Nadel, um robotische Einführungsschritte durchzuführen, jedoch zum Lösen des Griffs zwischen solchen Einführungsschritten, bis der nächste Einführungsschritt eingeleitet wird.

Aus der Druckschrift JP H02 71730 A ist zur Verringerung der Kosten einer medizinischen Einrichtung und ihres Platzbedarfs durch Bereitstellung einer Anordnung, bei der verschiedene Arten von Bildern durch Kombination einer Strahlungsquelle und einem Detektor, die jeweils von einem Paar von Roboterarmen eingespannt werden, bekannt. Zubehörteile verschiedener Arten von Bildgebungsvorrichtungen, wie z.B. ein Positionierungsmechanismus und dergleichen, können verwendet werden.

Aus der Druckschrift DE 10 2014 203 465 A1 ist eine Auswahleinheit, die dazu ausgebildet ist, auf Basis eines effektiven Strahlungsabsorptionsprofils eines Untersuchungsobjekts einen Strahlungsformfilter aus einer Mehrzahl von N Strahlungsformfiltern auszuwählen.

Aus der EP 2 302 644 A2 ist eine Motorsteuerung bekannt, welche die Antriebsgeschwindigkeit eines Motors zum Antrieb eines Filters bestimmt, so dass ein Flächenverschiebungszyklus des Filters mit einem Herzzyklus synchronisiert wird.

Es ist Aufgabe der Erfindung, eine Manipulatorvorrichtung zur automatischen Einrichtung eines Radiologiearbeitsplatzes, ein medizinisches Bildgebungsgerät und ein Verfahren dazu anzugeben, welche eine automatische Einrichtung eines Radiographiearbeitsplatzes ermöglichen.

Die Aufgabe wird erfindungsgemäß gelöst durch eine Manipulatorvorrichtung zur automatischen Einrichtung eines Radiologiearbeitsplatzes nach Anspruch 1, ein medizinisches Bildgebungsgerät nach Anspruch 13 und ein Verfahren nach Anspruch 14.

Die Erfindung betrifft eine Manipulatorvorrichtung zur automatischen Einrichtung eines Radiologiearbeitsplatzes eines Bildgebungssystems aufweisend eine Sensoreinheit und eine automatische Manipulatoreinheit. Die Sensoreinheit ist zum Lokalisieren einer mindestens einen beweglichen Komponente des Radiologiearbeitsplatzes ausgelegt. Die automatische Manipulatoreinheit ist zur mechanischen Aufnahme der Komponente und zur Positionierung der Komponente an einer für die Untersuchung vorgesehenen Position ausgelegt. Die Sensoreinheit ist zur Komponentenerkennung ausgelegt, und die Manipulatorvorrichtung ist zum Datenaustausch mit dem Bildgebungssystem ausgebildet, um untersuchungsspezifische Informationen zu erhalten, um eine spezielle, zum Bildgebungssystem gehörige Komponente auszuwählen.

Die Erfindung löst das Problem der Raumeinrichtung bzw. der Einrichtung eines Radiographiearbeitsplatzes so, dass an einer beispielsweise vollautomatisch verfahrbaren Untereinheit des Röntgensystems eine Manipulatoreinheit vorgesehen ist, die es ermöglicht, auch nicht-motorisierte bzw. nicht sinnvoll motorisierbare Komponenten des Röntgensystems oder/und des Raumes zu manipulieren. Das Röntgensystem ist bevorzugt ein Radiographiesystem.

Der Radiologiearbeitsplatz kann insbesondere als Radiographiearbeitsplatz ausgebildet sein. Der Radiologiearbeitsplatz umfasst ein insbesondere medizinisches Bildgebungssystem. Der Radiographiearbeitsplatz umfasst insbesondere ein Röntgenbasiertes Bildgebungssystem, beispielsweise ein Radiographiesystem oder ein anderes Röntgensystem. Die bewegliche Komponente ist insbesondere frei beweglich bzw. mobil.

Die automatische Manipulatoreinheit kann auch als Positionierungseinheit oder Bewegungseinheit bezeichnet werden. Die Manipulatoreinheit kann eine Steuer- oder eine Recheneinheit umfassen. Die Rechen- bzw. die Steuereinheit kann zumindest teilweise ein maschinelles Lernverfahren einsetzen. Dabei können Features zur Personenerkennung, zur Bewegungsvorhersage von Personen oder Gegenständen sowie zur Kollisionsvermeidung algorithmisch trainiert werden.

Maschinelles Lernen im Sinne der Anmeldung umfasst eine computer-implementierte Technik, bei der ein Algorithmus auf Basis von bestehenden Daten Muster bzw. Gesetzmäßigkeiten erkennt und unter Anwendung derselben in Bezug auf unbekannte, neue Daten eigenständig Lösungen ableitet. Voraussetzung für eine eigenständige Lösungsfindung ist eine Trainingsphase, in der ein Algorithmus des Maschinen-Lernens auf einen bekannten, definierten und zumeist sehr großen Datenbestand angewandt wird, um diejenigen Regeln bzw. Vorhersagen zu finden, die eine gewünschte Ausgabe bzw. ein gewünschtes Ergebnis erzielen. Das Training kann als überwachtes oder unüberwachtes Training ausgebildet sein, wobei in der ersten Variante dem Algorithmus Werte-Paare in Form von Eingabewerten und dazu gehörigen, korrekten Ausgabewerten präsentiert werden, wohingegen in der zweiten Variante der Algorithmus sich basierend auf den Eingabewerten eigenständig derart selber anpassen muss, dass er die korrekten Ausgabewerte liefert.

Besonders vorteilhaft ist der Algorithmus des Maschinen-Lernens als künstliches neuronales Netz ausgebildet. Ein künstliches neuronales Netz orientiert sich am Aufbau eines biologischen, neuronalen Netzes wie bspw. einem menschlichen Gehirn. Ein künstliches neuronales Netz umfasst zwischen einer Eingabe- und einer Ausgabeschicht bevorzugt eine Vielzahl von weiteren Schichten jeweils umfassend wenigstens einen Knoten. Jeder Knoten entspricht dabei einer Verarbeitungseinheit, analog einem biologischen Neuron. Knoten innerhalb einer Schicht des Netzes können über gerichtete Verbindungen (edges) mit Knoten anderer Schichten verbunden sein. Die Verbindungen definieren den Datenfluss innerhalb des Netzes. Jeder Knoten repräsentiert folglich eine Operation, die auf die Eingabedaten angewendet wird. Ferner verfügt jeder Knoten bzw. jede seiner Verbindungen über einen Gewichtungsparameter (weight). Über diesen Gewichtungsparameter wird der Einfluss bzw. die Wichtigkeit der Ausgabe eines Knotens als Eingabewert für einen Empfängerknoten definiert. In der Trainingsphase, die bevorzugt als überwachtes Lernen ausgeführt wird, ,lernt' das Künstliche Neuronale Netz anhand der Trainingsdaten die Gewichtungsparameter für alle Knoten bzw. Verbindungen und passt diese solange an, bis die Ausgabeschicht des Netzes die korrekten Ausgabewerte liefert.

Diese Vorgehensweise beruht ferner auf der Erkenntnis, dass ein trainierter Algorithmus des Maschinen-Lernens im Rahmen seines Trainings einen festen Zusammenhang zwischen Eingabewerten, hier in Form von Positionen oder Koordinaten und Eigenschaft der Person oder des Gegenstands (Benutzer, Patient, Objekt), Untersuchungsfortschritt oder ähnlichem, und Ausgabewerten, beispielsweise in Form von Bewegungspfaden, herstellt.

Besonders bevorzugt werden beide Schritte des erfindungsgemäßen Verfahrens mittels eines Algorithmus ausgeführt. Besonders geeignet sind hierfür Algorithmen des sogenannten Deep Learnings, bspw. in Form eines ,convolutional neural networks', auch faltendes neuronales Netz genannt. Mit anderen Worten wird gemäß dieser Ausführung mittels eines Algorithmus des Maschinen-Lernens zunächst eine Merkmalsextraktion durchgeführt, und anschließend eine sogenannte Klassifikation, wobei die identifizierten Merkmale einem Bewegungspfad o.ä. zugeordnet werden. Alternativ zu einem faltenden neuronalen Netz können auch Lange Kurzzeit-Gedächtnis (LSTM long shortterm memory) Netze oder rekurrente neuronale Netze (RNN) zum Einsatz kommen, welche im Gegensatz zu den vorher genannten rückwärts gerichtete Feedback-Schleifen innerhalb der versteckten Netzschichten aufweisen.

Gemäß einem Aspekt der Anmeldung kann ein nachfolgend beschriebenes künstliches neuronales Netz zum Einsatz kommen. Das neuronale Netz antwortet auf Eingabewerte zu einer Vielzahl von Eingangsknoten die angewendet werden, um eine oder eine Vielzahl von Ausgaben zu erzeugen. Das neuronale Netz lernt in diesem Ausführungsbeispiel, indem es die Gewichtungsfaktoren (weights) der einzelnen Knoten basierend auf Trainingsdaten anpasst. Mögliche Eingabewerte der Eingangsknoten können beispielsweise bestimmte Positionen oder Eigensachften als Annotationen vorheriger Ausgaben sein, die zuvor aus bestehenden Datensätzen extrahiert wurden. Alternativ kann das neuronale Netz ausgebildet sein, auch die Merkmalsextraktion durchzuführen. Beliebige andere Eingabewerte können zur Anwendung kommen. Das neuronale Netz gewichtet die Eingabewerte basierend auf dem Lernprozess. Die Ausgabewerte des neuronalen Netzes entsprechen bevorzugt einem ermittelten Bewegungspfad oder einer erkannten Person. Die Ausgabe kann über einen einzelnen oder eine Vielzahl von Ausgabeknoten erfolgen.

Das künstliche neuronale Netz umfasst bevorzugt eine versteckte Schicht, die eine Vielzahl von Knoten umfasst. Es können mehrere versteckte Schichten vorgesehen sein, wobei eine versteckte Schicht Ausgabewerte einer anderen versteckten Schicht als Eingabewerte verwendet. Die Knoten einer versteckten Schicht verrichten mathematische Operationen. Ein Ausgabewert eines Knotens $h_i$ entspricht dabei einer nichtlinearen Funktion f seiner Eingabewerte $x_i$ und der

Gewichtungsfaktoren $w_i$. Nach dem Erhalt von Eingabewerten $x_i$, führt ein Knoten $h_j$ eine Summierung einer mit den Gewichtungsfaktoren $w_i$ gewichteten Multiplikation jedes Eingabewerts $x_i$ durch, wie durch folgende Funktion bestimmt:

$$h_j \; = \; f\left(\sum_i x_i \cdot w_{ij}\right).$$

Insbesondere wird ein Ausgabewert eines Knotens $h_j$ als Funktion $f$ einer Knoten-Aktivierung, bspw. eine Sigmoidalfunktion oder eine lineare Rampenfunktion, gebildet. Die Ausgabewerte $h_j$ werden an den bzw. die Ausgabeknoten $o_j$ übertragen. Erneut wird eine Summierung einer gewichteten Multiplikation jedes Ausgabewertes $h_j$ als Funktion der Knoten-Aktivierung $f$ berechnet:

$$o_j \; = \; f\left(\sum_i h_i \cdot w'_{ij}\right).$$

Das neuronale Netz kann ein Feedforward neuronales Netz sein, bei dem alle Knoten die Ausgabewerte einer vorherigen Schicht in Form ihrer gewichteten Summe als Eingabewerte verarbeiten. Selbstredend können erfindungsgemäß auch andere neuronale Netztypen zum Einsatz kommen, bspw. Feedback-Netze, bei denen ein Eingabewert eines Knotens $h_j$ gleichzeitig auch sein Ausgabewert sein kann.

Das neuronale Netz kann mittels einer Methode des überwachten Lernens trainiert werden, um Muster zu erkennen. Eine bekannte Vorgehensweise ist die Back-Propagation, die für alle Ausführungsbeispiele der Erfindung angewandt werden kann. Während des Trainings wird das neuronale Netz auf Trainings-Eingabewerten angewandt und muss entsprechende, vorher bekannte Ausgabewerte erzeugen. Iterativ werden mittlere quadratische Fehler (mean square error - "MSE") zwischen berechneten und erwarteten Ausgabewerten berechnet und einzelne Gewichtungsfaktoren so lange angepasst, bis die Abweichung zwischen berechneten und erwarteten Ausgabewerten unterhalb einer vorbestimmten Schwelle liegt.

Die automatische Manipulatoreinheit ist zur mechanischen Aufnahme der Komponente ausgebildet, d.h. die Manipulatoreinheit kann die Komponente beispielsweise greifen bzw. eine lösbare mechanische Verbindung herstellen. Die Manipulatoreinheit kann eine Manipulatorschnittstelle aufweisen. Die Manipulatoreinheit ist zur Positionierung der Komponente an einer für die Untersuchung vorgesehenen Position ausgelegt, d.h. die Manipulatoreinheit kann die Position der Komponente manipulieren bzw. die Komponente zu einer anderen Position bewegen. Die Positionierung kann auch als Manipulation oder Bewegung bezeichnet werden. Die automatische Manipulatoreinheit kann sich insbesondere vollständig motorisiert bewegen. Die Bewegung der automatischen Manipulatoreinheit kann insbesondere gesteuert werden. Es kann eine Ausrüstung einer motorisiert verfahrbaren Teileinheit des Radiographiesystems mit einer Manipulatorvorrichtung bzw. Manipulatoreinheit erfolgen.

Weiter ist eine Sensoreinheit vorgesehen, die es ermöglicht, die nicht-motorisierten Komponenten aufzufinden bzw. sicher und kollisionsfrei, um beweglich Gegenstände oder bewegliche Personen zu manövrieren. Die Manipulatorvorrichtung umfasst eine Sensoreinrichtung, die es ermöglicht, die nichtmotorisch verfahrbaren Komponenten zu erkennen.

Die Vorteile der Erfindung bestehen darin, dass sich nun mindestens eine Komponente oder im Wesentlichen alle Komponenten eines Untersuchungsraumes bzw. der Radiologiearbeitsplatzes auf die kommende Untersuchung vorbereiten lassen und nicht nur die Komponenten, die selbst über eine Motorisierung verfügen. Vorteilhaft können nicht-motorisierte Komponenten an die für die Untersuchung nötige Position mittels der automatischen Manipulatoreinheit bzw. der Manipulatorvorrichtung gebracht werden.

In einer besonders vorteilhaften Ausgestaltung kann ein Systemdesign erreicht werden, welches eine Untersuchung komplett ohne menschliches Zutun bzw. ohne manuelle Vorbereitungsschritte vorbereiten kann. Ein lokaler Systembenutzer kann in dieser besonders vorteilhaften Ausgestaltung nicht mehr notwendig sein. Ein Benutzer kann generell dann nur noch zur Erfüllung regulatorischer Anforderungen bei der Auslösung von Röntgenstrahlung notwendig sein, dies kann ggf. auch aus der Ferne über eine Remote-Verbindung erfolgen. Vorteilhaft kann die Wirtschaftlichkeit von Untersuchungen erhöht werden. Zusätzlich kann die Verfügbarkeit von Röntgensystemen insbesondere in ländlichen Regionen von Schwellenländern erhöht werden. Vorteilhaft können Herstellungskosten gespart werden, beispielsweise hinsichtlich der Anzahl nötiger, festverbauter digitaler Röntgendetektoren. Beispielsweise können bisher zur Erreichung einer maximalen Workfloweffizienz bis zu zwei Röntgendetektoren fest in einem Rasterwandgerät und einem Patiententisch eines Radiographiesystems verbaut werden und zusätzlich ein oder mehrere mobile Röntgendetektoren verwendet werden. Dies ist ein erheblicher Kostennachteil gegenüber der Verwendung desselben Detektors für alle Aufnahmen. Die Erfindung ermöglicht die im Wesentlichen gleiche Workfloweffizienz der festverbauten Röntgendetektoren mit nur einem mobilen Detektor, da die Manipulatorvorrichtung beispielsweise noch bevor ein Benutzer oder Patient der Untersuchungsraum betritt, den mobilen Röntgendetektor bei Bedarf bereits vom Patiententisch zum Rasterwandgerät oder in eine freie Position bewegt hat. Gegenüber der manuellen Lösung durch den Benutzer ergibt sich der Vorteil, dass die Wahrscheinlichkeit der Beschädigung des mobilen Röntgendetektors durch den automatischen Transport minimiert werden kann. Das ist insofern sehr relevant, da die Beschädigung eines freien bzw. mobilen Röntgendetektors derzeit eine der häufigsten Fehlerursachen bei Radiographiesystemen darstellt. Weiterhin kann es durch die Unterstützung der Manipulatorvorrichtung bei der Positionierung, insbesondere des Röntgendetektors, auch möglich sein, die Qualität der Untersuchung zu verbessern und Fehler durch eine ungenaue Positionierung zu vermeiden.

Ein erfindungsgemäß ausgestattetes Radiographiesystem ermöglicht es dem Benutzer, in seinem Arbeitsablauf Zeit für die Raumvorbereitung und dadurch Kosten zu sparen. Vorteilhaft kann ein solches Radiographiesystem für den Kunden attraktiver ausgestaltet sein. Beispielsweise können Personalkosten bzw.- zeiten eingespart werden. Besonders vorteilhaft kann die Kostenerhöhung durch die Manipulatorvorrichtung geringer sein als sein als die eingesparten Personalkosten, so dass das Bildgebungssystem günstiger betrieben werden kann.

Gemäß einem Aspekt der Erfindung ist die, insbesondere nicht-motorisierte, Komponente ein (digitaler) Röntgendetektor, ein Positionierungshilfsmittel, ein Raster oder eine Filtereinheit. Der Röntgendetektor kann insbesondere ein mobiler Röntgendetektor sein. Das Positionierungshilfsmittel kann beispielsweise eine Vorrichtung zur Stabilisierung der Patientenposition sein. Das Positionierungshilfsmittel kann beispielsweise eine Haltevorrichtung, beispielsweise ein Griff, oder eine Lagerungshilfe, beispielsweise ein Lagerungskissen, sein.

Zur Raumvorbereitung bzw. Einrichtung eines Radiographiearbeitsplatzes können die Positionierung von Röntgenquelle, Röntgendetektor, Patiententisch oder/und Rasterwandgerät zählen. Zudem kann die Einrichtung auch die Bereitstellung von Positionierungshilfsmitteln oder/und das Vorbereiten des Röntgendetektors umfassen. Das Vorbereiten des Röntgendetektors kann beispielsweise das Anbringen oder das Einlegen eines Rasters und/oder das Einschieben eines mobilen bzw. frei positionierbaren Röntgendetektor in den Patiententisch oder das Rasterwandgerät umfassen.

Die Manipulatoreinheit kann derart ausgestaltet sein, dass sich damit ein mobiler Röntgendetektor und/oder ein Raster mechanisch aufnehmen lassen und in den Patiententisch, das Rasterwandgerät oder allgemein im Raum, beispielsweise auf den Patiententisch, auf den Boden oder ähnliches, oder insbesondere ein Raster auf einem mobilen Röntgendetektor positionieren oder/und befestigen lassen. Zusätzlich kann die Manipulatoreinheit dahingehend ausgestaltet sein, dass sie das Positionierungshilfsmittel, beispielsweise ein Schaumkeil, an die benötigte Position bringen kann. Vorteilhaft kann der Radiologiearbeitsplatz für die Untersuchung automatisch vorbereitet werden.

Gemäß einem Aspekt der Erfindung kann eine Aufbewahrungseinheit für mindestens eine Komponente an der Manipulatorvorrichtung oder dem Bildgebungssystem vorgesehen sein, welche von einer vollautomatisch beweglichen Untereinheit des Bildgebungssytems bzw. der Manipulatoreinheit angefahren werden kann, um eine Manipulation der Komponente zu bewirken.

Beispielsweise kann eine Filtereinheit ein Vorsatzfilter sein. Für den Wechsel eines Vorsatzfilters vor der am Strahlerkopf der Röntgenquelle befestigten Strahlerblende können verschiedene Vorsatzfilter in der Aufbewahrungseinheit vorgehalten werden. Bisher können verschiedene Möglichkeiten der Filterung der Röntgenstrahlung in die Strahlerblende integriert sein und automatisch und manuell von außen anwählbar sein, trotzdem gibt es nach wie vor Anwendungen, bei denen ein Vorsatzfilter manuell außen an der Blende, typischerweise durch das Einschieben in dafür vorgesehene Schlitze, angebracht wird. Dies kann automatisiert werden, indem eine Aufbewahrungseinheit für einen oder mehrere Vorsatzfilter so ausgestaltet ist, dass der Strahlerkopf diese so anfährt, dass der gelagerte Vorsatzfilter in eine Vorrichtung am Strahlerkopf eingeschoben wird. Alternativ kann der Vorsatzfilter auch wieder aus dieser Vorrichtung herausgezogen und dabei entfernt werden, wenn er von der Aufbewahrungseinheit festgehalten wird, während sich der Strahlerkopf entfernt. Dieses ist sowohl mit zusätzlicher Sensorik und Aktuatoren in der Strahlerblende und/oder in der Aufbewahrungseinheit möglich, als auch ganz ohne zusätzliche Sensoren und Aktoren durch die mechanische Ausgestaltung der Aufbewahrungseinheit.

Gemäß einem Aspekt der Erfindung umfasst die Sensoreinheit eine Kameraeinheit. Die Sensoreinheit kann eine oder mehrere 2D- oder 3D-Kameras umfassen, die den Raum erfassen und die Position der nicht-motorisierten Komponente im Raum allgemein erkennen können. Die Sensoreinheit kann beispielsweise die aktuelle Position des mobilen Röntgendetektors im Raum erkennen und es dadurch der Manipulatoreinheit erlauben, den mobilen Röntgendetektor von einer beliebigen Position aus in oder auf den Patiententisch, in das Rasterwandgerät oder generell an die gewünschte Position im Raum zu verbringen. Diese Sensoreinheit kann dafür genutzt werden, nach einer Untersuchung den Untersuchungsraum bzw. den Radiologiearbeitsplatz aufzuräumen und beispielsweise die Positionierungshilfsmittel wieder an den dafür vorgesehenen Platz bzw. zur Aufbewahrungseinheit zu verbringen. Die Anwendung von 2D- oder 3D-Kameras als Sensoreinheit kann dadurch vereinfacht werden, dass an den zu erkennenden nicht-motorisierten Elementen optische Marker in Form von (retro-)reflektierenden Mustern, QR-Codes o.ä. angebracht sind, die die Sensoreinheit erkennen und zuordnen kann.

Die Sensoreinheit kann so ausgestaltet sein, dass sie die Aufnahme einer nicht-motorisierte Komponente detektieren bzw. lokalisieren kann und zudem insbesondere zusätzlich auch die Art der aufgenommenen Komponente bestimmen kann. Eine derartige Sensoreinheit kann es erforderlich machen, dass die Position der nicht-motorisierten Komponente mindestens ungefähr bekannt ist, beispielsweise können sich die Raster in einer Rasteraufbewahrungseinheit befinden und von der Manipulatoreinheit von dort entnommen und auch wieder dorthin verbracht werden. Eine solche Rasteraufbewahrungseinheit kann vorzugsweise an der Decke angebracht werden, damit sie im normalen Arbeitsablauf nicht im Weg ist. Eine andere sinnvolle Positionierung kann an der Wand sein, was einen manuellen Austausch des Rasters nach wie vor erlauben würde, da an der Decke die Aufbewahrungseinheit vom Benutzer wahrscheinlich nicht erreichbar wäre. Zusätzlich oder alternativ zu einer Rasteraufbewahrungseinheit können auch Aufbewahrungseinheiten für Positionierungshilfen, weitere Zubehörteile wie beispielsweise am Bildgebungssystem anzubringende Griffe vorgesehen sein. Vorteilhaft kann die Sensoreinheit die Komponente erkennen und deren Position zuverlässig lokalisieren bzw. während des Bewegens den Weg lokalisieren.

Gemäß einem Aspekt der Erfindung ist die Sensoreinheit ferner zur Personenerkennung ausgebildet. Insbesondere eine Sensoreinheit in Form einer 2D- bzw. 3D-Kamera kann zur Personenerkennung ausgebildet sein. Die Sensoreinheit kann beispielsweise Benutzer wiedererkennen bzw. Patienten beispielsweise basierend auf einem Foto in der Patientenakte erkennen. In einer besonders vorteilhaften Ausgestaltung kann die Sensoreinheit eine Bewegungsvorhersage für die im Untersuchungsraum befindlichen Personen erstellen. Für die Bewegungsvorhersage kann insbesondere ein Verfahren des maschinellen Lernens verwendet werden, für welches als Trainingsdaten bekannte Bewegungsprofile vorhergehender Untersuchungen genutzt werden. Vorteilhaft können Kollisionen der Manipulatorvorrichtung mit Personen vermieden werden. Vorteilhaft kann eine besonders geeignete Position für die Komponente während der Untersuchung bestimmt werden. Die Sensoreinheit kann derart ausgebildet sein, um einen registrierten Benutzer, beispielsweise MTRA, von einem Patienten zu unterscheiden. Dazu können beispielsweise optische oder akustische biometrische Merkmale genutzt werden.

Gemäß einem Aspekt der Erfindung umfasst die Sensoreinheit eine Positionselektronikeinheit. Die Sensoreinheit kann eine Positionselektronikeinheit umfassen, wobei die Positionselektronikeinheit Signale einer aktiven Elektronik, die an den nicht-motorisierten Komponenten angebracht wird, empfängt. Die aktive Elektronik kann beispielsweise als Lagesensor ausgebildet sein. Beispielsweise kann die Position der Komponente im Untersuchungsraum oder die Ausrichtung der Komponente im Untersuchungsraum bestimmt und drahtlos an die Positionselektronikeinheit bzw. die Manipulatorvorrichtung bzw. die Sensoreinheit übermittelt werden. Vorteilhaft kann die Position der Komponente einfach bestimmt werden. Vorteilhaft kann die Komponente einfach lokalisiert werden.

Gemäß einem Aspekt der Erfindung ist die Sensoreinheit ferner zur Komponentenerkennung ausgelegt. Die Sensoreinheit kann die Komponente beispielsweise mittels einer optischen Markierung, beispielsweise ein QR-Code, und einer Kameraeinheit erkennen. Die Sensoreinheit kann die Komponente mittels einer mechanischen Codierung, beispielsweise Stiften und Vertiefungen an einer Kontaktfläche, erkennen. Die Sensoreinheit kann die Komponente mittels elektromagnetischer Strahlung erkennen, beispielsweise mittels RFID oder Ultraschall. Vorteilhaft kann die Manipulatorvorrichtung die Komponente automatisch erkennen und die richtige Komponente auswählen.

Gemäß einem Aspekt der Erfindung ist die Sensoreinheit ferner zur Kollisionsvermeidung ausgelegt. Die Kollisionsvermeidung kann beispielsweise auf einem maschinellen Lernverfahren basieren, wobei Bewegungsabläufe früherer Vorbereitungen für den Radiologiearbeitsplatz als Trainingsdaten verwendet werden können. Die Sensoreinheit kann beispielsweise Berührungs- oder Radarsensoren umfassen. Vorteilhaft können insbesondere Kollisionen der Manipulatorvorrichtung mit Gegenständen oder Personen im Untersuchungsraum vermieden werden.

Gemäß einem Aspekt der Erfindung ist die Manipulatoreinheit eine vollautomatisch verfahrbare Untereinheit des Röntgensystems, eine Röntgenquellenbewegungseinheit oder eine Röntgendetektorbewegungseinheit. Die vollautomatisch verfahrbare Untereinheit kann insbesondere in Form einer Röntgenquellenbewegungseinheit oder einer Röntgendetektorbewegungseinheit ausgebildet sein. Die Röntgendetektorbewegungseinheit kann ähnlich einer Röntgenquellenbewegungseinheit ausgestaltet sein, wobei statt einer Röntgenquelle ein Röntgendetektor montiert ist und ggf. eine geringere Tragkraft ausreichend ist. Die Manipulatoreinheit kann als Robotereinheit bezeichnet werden. Die Manipulatorvorrichtung kann insbesondere vom Bildgebungssystem umfasst sein. Alternativ kann die Manipulatorvorrichtung vom Bildgebungssystem getrennt ausgestaltet sein. Die vollautomatisch verfahrbare Untereinheit kann beispielsweise der Strahlerkopf bzw. die Röntgenquelle oder insbesondere bei einem sogenannten Twin-Robotic-System der Detektorarm sein. Der Detektorarm kann eine, beispielsweise deckenmontierte, Befestigungs- und Bewegungseinheit sein, welche dazu ausgelegt ist, einen daran befestigten Röntgendetektor für eine Aufnahme bzw. Untersuchung zu positionieren. Die Röntgenquellenbewegungseinheit kann als U-Arm-Erweiterung am Deckenstativ der Röntgenquelle ausgebildet sein. Die Röntgenquellenbewegungseinheit kann 6 Bewegungsfreiheitsgrade aufweisen. Die Manipulatorvorrichtung bzw. die Manipulatoreinheit kann an der Röntgenquellenbewegungseinheit temporär oder permanent fixiert sein. Vorteilhaft kann die bereits vorhandenen Beweglichkeit der Röntgenquellenbewegungseinheit von der Manipulatoreinheit genutzt werden.

Die Röntgendetektorbewegungseinheit kann ein Detektorarm für den Röntgendetektor sein, welcher an der Raumdecke, wie auch das Deckenstativ der Röntgenquelle, montiert ist. Die Röntgendetektorbewegungseinheit ermöglicht eine vollautomatische Bewegung des Röntgendetektors. Die Röntgendetektorbewegungseinheit kann beispielsweise in robotischen Radiographiesystemen vorgesehen sein. Die Bewegungseinheit des Röntgendetektors, d.h. die Röntgendetektorbewegungseinheit, kann mit der Manipulatorvorrichtung oder der Manipulatoreinheit erweitert werden. Die Manipulatoreinheit kann zusätzlich an der Röntgendetektorbewegungseinheit bzw. der Röntgenquellenbewegungseinheit befestigt werden.

Die Manipulatoreinheit kann beispielsweise statt dem Röntgendetektor an einer (Röntgendetektor-) Bewegungseinheit befestigt werden, zumindest während der Vorbereitung des Radiologiearbeitsplatzes. Die Manipulatoreinheit kann statt des Röntgendetektors eine andere nicht-motorisierte Komponente an einer Manipulatorschnittstelle oder an mehreren Manipulatorschnittstellen aufnehmen. Die Röntgendetektorbewegungseinheit kann als Manipulatoreinheit ausgebildet sein. Bei einem robotischen Radiographiesystem kann die Manipulatoreinheit zur Aufnahme des Röntgendetektors optimiert sein. Der Röntgendetektor kann je nach Bedarf vom Röntgendetektorbewegungseinheit bzw. der Manipulatoreinheit entweder direkt an der Röntgendetektorbewegungseinheit verwendet werden oder von der Manipulatoreinheit in einen Patiententisch bzw. ein Rasterwandgerät verbracht werden. Vorteilhaft kann sowohl die Manipulatoreinheit als auch die Sensoreinheit sehr einfach ausgestaltet sein, da jeweils nur der Röntgendetektor bewegt bzw. an einer bereits bekannten Position erkannt werden muss. Es kann vorteilhaft nur eine einzige Manipulatorschnittstelle nötig und ausgebildet sein.

Ein weiteres Ausführungsbeispiel ist ein sogenanntes "U-Arm System". Der (Standard-)Strahler bzw. die Röntgenquelle am Deckenstativ bzw. an der Röntgenquellenbewegungseinheit kann durch ein ausfahrbares Teleskop oder einen ansteckbaren Ausleger mit einem Detektorhalter bzw. einer Manipulatoreinheit in Form einer Halterung für einen Röntgendetektor zu einem U-Arm-System erweitert werden. Somit kann ein Deckenstativ-System zu einem U-Armsystem bei Bedarf erweitert werden. Vorteilhaft kann die gesamte Flexibilität erhöht werden. Vorteilhaft können die Eigenschaften des klassischen Deckenstativ-Systems mit dem des U-Arm-Systems kombiniert werden. Eine vorteilhafte Ausgestaltung kann das Wandstativ bzw. das Rasterwandgerät als Parkposition des Auslegers bzw. der Manipulatorvorrichtung bzw. der Manipulatorenheit nutzen. Dadurch kann die Nutzung des Röntgendetektors in beiden Anwendungen, d.h. dem Deckenstativ-System und dem U-Arm-System, vorteilhaft ermöglicht werden. Beispielsweise kann der Röntgendetektor mittels der (Röntgenquellen-)Bewegungseinheit und der Manipulatoreinheit des U-Arm-Systems automatisch in ein Rasterwandgerät verbracht werden und der Rest des U-Arms mit der Röntgenquelle in eine weiter entfernte Position gebracht werden, so dass sich große Röntgenquellen-Röntgendetektor-Abstände, beispielsweise 3 m, erzielen lassen, selbst wenn die U-Arm-Mechanik nur auf kleinere Röntgenquellen-Röntgendetektor-Abstände, beispielsweise bis zu 1,80 m, ausgelegt ist. Vorteilhaft kann eine größere Anwendungsflexibilität für ein Radiographiesystem erreicht werden. Vorteilhaft kann ein größerer Röntgenquellen-Röntgendetektor-Abstand ermöglicht werden.

Die Manipulatorvorrichtung kann alternativ als dedizierter separater Roboter für die Raum- bzw. Radiologiearbeitsplatzvorbereitung ausgebildet sein.

Gemäß einem Aspekt der Erfindung ist die Manipulatorvorrichtung eine Drohneneinheit. Die Drohneneinheit ist dazu ausgelegt, Teile der Raumvorbereitung patientenspezifisch oder untersuchungsspezifisch zu übernehmen. Ein Datenaustausch der Manipulatoreinheit oder der Manipulatorvorrichtung mit dem medizinischen Bildgebungssystem, beispielsweise dem Radiographiesystem, ist ausgebildet, um beispielsweise Informationen über die geplante klinische Prozedur bzw. Untersuchung, die Mobilität des Patienten oder andere patienten- oder untersuchungsspezifische Informationen zu erhalten.

Die Sensoreinheit kann ausgebildet sein, um statische und mobile Einrichtungsgegenstände in der Umgebung als auch sich bewegende Personen zu identifizieren. Beispielsweise kann die Sensoreinheit 2D-/3D-optische Sensoren, Radar, Sonar oder andere Positionsbestimmungsvorrichtungen verwendet werden. Die Sensoreinheit kann ausgebildet sein, um spezielle zum Bildgebungssystem gehörige Komponente sicher und eindeutig zu identifizieren. Insbesondere kann die Sensoreinheit eine Erkennung von einer Auswahl aus den folgenden Komponenten ermöglichen: mobile Röntgendetektoren, (Detektor-)Raster, Vorsatzfilter für die Strahlerblende und andere Zubehörteile wie Positionierungshilfen, Einweg-Papierrollen zur Hygiene, Vorsatzblendenraster für SPECT-Anwendungen oder Lokalspulen für Magnetresonanztomographen. Die Identifikation der Komponente kann beispielsweise elektromagnetisch über RFID-Tags oder optisch über QR-Codes erfolgen. Die Sensoreinheit kann ausgebildet sein, um die nicht motorisierten Elemente aufzufinden und/oder sicher und kollisionsfrei um mobile Gegenstände oder mobile Personen zu manövrieren.

Die Manipulatorvorrichtung kann die Komponente nach Identifikation oder/und Lokalisation aufnehmen. Die Manipulatorvorrichtung kann die Komponente durch die Luft an einen gewünschten Zielort transportieren. Die Komponente kann an der Zielposition mit anderen Komponenten, beispielsweise ein Röntgendetektor mit einem Raster oder ein Röntgendetektor mit einer Lagerungshilfe, kombiniert werden.

Die Drohneneinheit bzw. die Manipulatorvorrichtung kann beispielsweise einen mobilen Röntgendetektor selbst halten, um ihn in einer fliegenden Position für eine Untersuchung zu halten bzw. positionieren. Die Drohneneinheit bzw. die Manipulatorvorrichtung kann beispielsweise einen mobilen Röntgendetektor in einer komplexen Positionierung bzw. Position stabilisieren und dynamisch an die Position bzw. die Lage des Patienten anpassen.

Vorteilhaft sinken die Herstellungskosten für Drohnentechnologie. Vorteilhaft weist die Drohneneinheit bzw. die Manipulatorvorrichtung in Form einer Drohne wesentlich höhere bzw. mehr Freiheitsgrade auf.

Gemäß einem Aspekt der Anmeldung ist eine Parkeinheit zum Aufbewahren und Laden der Drohneneinheit am Radiologiearbeitsplatz oder im Untersuchungsraum oder am Bildgebungssystem vorgesehen. Die Drohneneinheit kann in der Parkeinheit bzw. der Ladestation im Untersuchungsraum eingedockt verbleiben und diese beispielsweise nur zum Einrichten des Radiologiearbeitsplatzes verlassen. Vorteilhaft ist die Drohneneinheit bzw. die Manipulatorvorrichtung verwahrt, so dass Kollisionen bzw. Störungen im Untersuchungsablauf vermieden werden können.

Gemäß einem Aspekt der Erfindung umfasst die Manipulatoreinheit eine Desinfektionseinheit. Die Desinfektionseinheit kann eine Sprüheinrichtung für ein Desinfektionsmittel oder eine Auftragungseinheit für ein Reinigungsmittel umfassen. Die Desinfektionseinheit kann beispielsweise an der Manipulatorschnittstelle aufgenommen werden. Alternativ kann die Desinfektionseinheit direkt an der Manipulatoreinheit befestigt sein. Mittels der Desinfektionseinheit kann das Bildgebungssystem und/oder die Manipulatoreinheit und/oder Komponenten desinfizieren bzw. reinigen. Ferner kann eine Reinigungseinheit von der Desinfektionseinheit umfasst sein, welche das Desinfektionsmittel oder/und das Reinigungsmittel verteilen oder/und abwischen kann. Vorteilhaft kann eine Reinigung oder Desinfektion nach der Untersuchung von der Desinfektionseinheit durchgeführt werden.

Gemäß einem Aspekt der Erfindung umfasst die Manipulatoreinheit eine Patientenhalteeinheit. Die Patientenhalteeinheit kann die Manipulatoreinheit sein, ggf. kann eine Lagerungshilfe o.ä. an der Manipulatorschnittstelle aufgenommen werden. Die Manipulatoreinheit kann nachdem der Benutzer den Patienten positioniert hat, den Patienten in der entsprechenden Position, insbesondere sanft, halten. Die Manipulatoreinheit kann den Patienten vor, während und nach der Untersuchung unterstützen, beispielsweise bis der Benutzer den Raum verlassen hat, die Strahlung ausgelöst hat und die Untersuchung abgeschlossen ist. Die Manipulatoreinheit kann als Robotereinheit ausgebildet sein. Dafür kann zusätzlich eine zweite Manipulatoreinheit vorgesehen sein, wie beispielsweise bei einem Twin-robotischen System. Alternativ kann die Manipulatoreinheit über genügend Freiheitsgrade verfügen, um den Patienten zu halten während die Röntgenquelle, an deren Röntgenquellenbewegungseinheit die Manipulatoreinheit beispielsweise befestigt ist, auf einen Untersuchungsabstand verfährt, um das Bild aufzunehmen. Vorteilhaft kann eine verbesserte Bildqualität erreicht werden.

Zudem kann die Manipulatoreinheit mit hinreichend Tragkaft ausgestattet sein, um den Benutzer dabei zu unterstützen, immobile Patienten z.B. auf den Patiententisch zu heben, also als eine Art Kran zu fungieren, um den Patienten umzulagern. Dabei können weitere Komponenten, insbesondere Lagerungshilfen nötig sein wie beispielsweise eine Trage oder ein Tuch bzw. Netz, das mit Tragschlaufen versehen ist, die von der Manipulatoreinheit gegriffen bzw. mechanisch aufgenommen werden können.

Alternativ kann die Manipulatorvorrichtung eine Drohneneinheit mit einer Patientenhalteeinheit umfassen. Die Manipulatoreinheit kann dafür ausgestaltet sein, um als Haltehilfe für Patienten zu agieren, beispielsweise, um dem Patienten zu helfen die Arme nach oben zu strecken und in dieser Position zu halten. Die Manipulatoreinheit kann derart ausgestaltet sein, um den Patienten in schwierigen Positionen zu stützen. Vorteilhaft kann der Benutzer bei der Patientenpositionierung unterstützt werden.

Gemäß einem Aspekt der Erfindung sind mehrere Manipulatoreinheiten vorgesehen, welche kollaborativ miteinander agieren können. Beispielsweise können mehrere Drohneneinheiten im Raum kollaborativ zusammenarbeiten können, beispielsweise um vorteilhaft Zubehörteile bzw. Komponenten kombinieren zu können.

Gemäß einem Aspekt der Erfindung weist die Manipulatorvorrichtung ferner eine Anzeigeeinheit zur Patientenpositionierung auf. Es kann Anzeigeeinheit in Form einer Beleuchtungseinheit, eines Bildschirms oder einer Videoeinheit zum **Ab**spielen eines Videos oder einer Audioeinheit zum Abspielen eines akustischen Signals an der Manipulatorvorrichtung vorgesehen sein, welche den Patienten zu einer selbständigen Positionierung instruieren.

Die Anzeigeeinheit kann als Leuchtmittel auf dem Patiententisch oder auf dem Rasterwandgerät ausgebildet sein. Es können für bestimmte Untersuchungen relevante "Points of Interest" angezeigt werden. Alternativ kann beispielsweise ein deckengehängter Beamer oder Laser von der Manipulatoreinrichtung umfasst sein, welche die entsprechende Komponente oder Raumregionen anleuchten. Alternativ kann die Anzeigeeinheit separat ausgebildet sein.

Nachdem der Manipulatoreinheit den Untersuchungsraum beispielsweise mit Positionierhilfe, Röntgendetektor, Raster oder einer anderen Komponente vorbereitet hat, kann ein Patient, insbesondere ohne Benutzer, in den Raum gelassen werden, beispielsweise ausgelöst durch ein Kommando via Video, Ansage oder Avatar, und beispielsweise von den Lichtelementen als Anzeigeeinheit an den Ort bzw. die Position der Untersuchung geführt werden.

Der Patient kann zunächst mittels eines Tutorialvideos oder einem Avatar oder einer Audioansage instruiert werden, wie er sich selbst positionieren soll. Die Kommandos zur Positionierung können beispielsweise "aufrecht stellen, Gesicht zur Wand, Arme seitlich angewinkelt" lauten. Anschließend kann die Manipulatorvorrichtung zusätzlich den Patienten vorsichtig in seiner selbstgewählten bzw. selbsteingenommen Positionierung korrigieren und bei Bedarf beispielsweise näher an das Rasterwandgerät führen oder die Arme stärker anwinkeln bis die Haltung des Patienten von der Sensoreinheit, welche beispielsweise auch die Manipulatorvorrichtung bzw. die Manipulatoreinheit oder die Drohneneinheit leitet, als korrekt erkannt wurde. Vorteilhaft kann eine verbesserte Patientenpositionierung erreicht werden. Vorteilhaft kann die Bildqualität gesteigert werden.

Die Erfindung betrifft ferner ein medizinisches Bildgebungssystem aufweisend die erfindungsgemäße Manipulatorvorrichtung. Das medizinische Bildgebungssystem kann insbesondere als Röntgen-/Radiographiesystem ausgestaltet sein. Das medizinische Bildgebungssystem kann als SPECT-System ausgestaltet sein, eine bevorzugte Komponente ist in diesem Fall ein Kollimator. Das medizinische Bildgebungssystem kann als Magnetresonanztomograph ausgestaltet sein, eine bevorzugte Komponente ist in diesem Fall eine Spule. Das medizinische Bildgebungssystem kann als Computertomographiesystem, Fluoroskopiesystem oder Angiographiesystem ausgestaltet sein. Die Vorteile der erfindungsgemäßen Manipulatorvorrichtung können vorteilhaft auf das medizinische Bildgebungssystem übertragen werden.

Die Erfindung betrifft ferner ein Verfahren zur automatischen Einrichtung eines Radiologiearbeitsplatzes eines erfindungsgemäßen Bildgebungssystems. Das Verfahren weist die folgenden Schritte, insbesondere in der genannten Reihenfolge, auf: Lokalisieren, mechanisches Aufnehmen und Positionieren. Im Schritt des Lokalisierens wird mindestens einer Komponente des Radiologiearbeitsplatzes lokalisiert. Im Schritt des mechanischen Aufnehmens wird die Komponente mittels einer automatischen Manipulatoreinheit mechanisch aufgenommen. Im Schritt des Positionierens wird die Komponente an einer für die Untersuchung vorgesehenen Position mittels der automatischen Manipulatoreinheit positioniert. Die Merkmale der erfindungsgemäßen Vorrichtungen können auf das erfindungsgemäße Verfahren übertragen werden.

Nachfolgend werden Ausführungsbeispiele der Erfindung anhand von Zeichnungen näher erläutert. Hierbei zeigt:

FIG 1 eine schematische Darstellung eines erfindungsgemäßen Bildgebungssystems in einer ersten Ausführungsform;
FIG 2 eine schematische Darstellung eines erfindungsgemäßen Bildgebungssystems in einer zweiten Ausführungsform; und
FIG 3 eine schematische Darstellung eines erfindungsgemäßen Verfahrens.

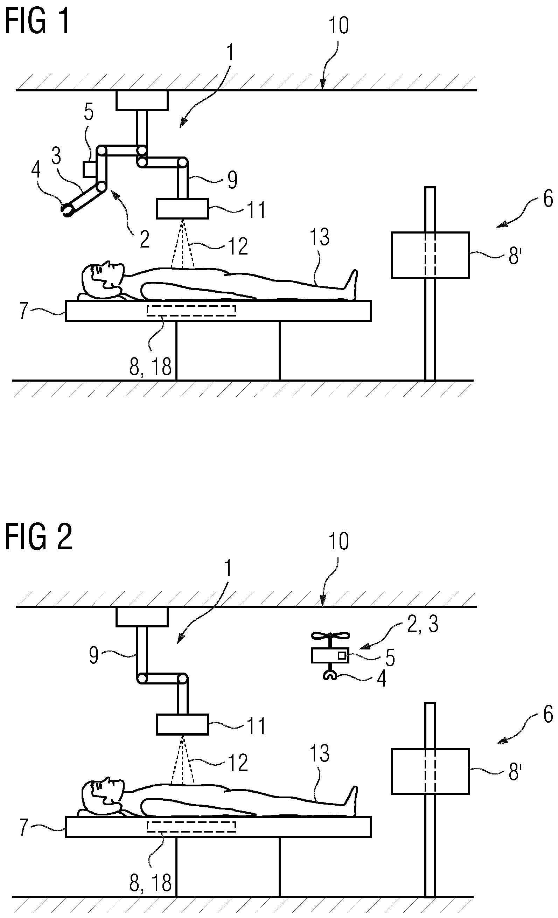

Die Fig. 1 zeigt eine beispielhafte Ausführung des erfindungsgemäßen Bildgebungssystems 1 in einer ersten Ausführungsform. Das Bildgebungssystem 1 ist ein Radiographiesystem in einem Untersuchungsraum 10, welcher einen Radiographiearbeitsplatz umfasst. Das Bildgebungssystem 1 weist eine Röntgenquelle 11 an einer Röntgenquellenbewegungseinheit 9 auf. Die Röntgenquellenbewegungseinheit 9 ist ein Deckenstativ. Die Röntgenquelle 11 sendet Röntgenstrahlen 12 in Richtung des Röntgendetektors in einer ersten Röntgendetektorposition 8 aus, wobei der Patient 13 auf dem Patiententisch 7 zwischen der Röntgenquelle 11 und dem Röntgendetektor in der ersten Röntgendetektorposition 8 angeordnet ist. Der Röntgendetektor ist die Komponente 18. Die ersten Röntgendetektorposition 8 ist als Einschubfach im Patiententisch 7 ausgebildet. Das Rasterwandgerät 6 weist eine zweite, alternative Röntgendetektorposition 8' auf. Der Röntgendetektor ist ein mobiler Röntgendetektor, der in der ersten Röntgendetektorposition 8, der zweiten Röntgendetektorposition 8' und als freier Röntgendetektor beispielsweise auf dem Patiententisch 7 verwendet werden kann. Es können zudem weitere Komponenten wie ein Positionierungshilfsmittel, angedeutet durch das Kissen unterhalb des Patientenkopfes, ein Raster am Röntgendetektor oder/und eine Filtereinheit an der Röntgenquelle 11 von der Manipulatorvorrichtung 2 positioniert werden. Die Sensoreinheit 5 ist bevorzugt als Kameraeinheit ausgebildet.

Die Manipulatorvorrichtung 2 ist am Röntgenquellenbewegungsarm 9 ausgebildet. Die Manipulatorvorrichtung 2 umfasst eine Manipulatoreinheit 3 mit einer Schnittstelle 4. Die Manipulatoreinheit umfasst ferner eine Sensoreinheit 5.

Die Fig. 2 zeigt eine beispielhafte Ausführung eines erfindungsgemäßen Bildgebungssystems 1 in einer zweiten Ausführungsform. Die Manipulatorvorrichtung 2 ist als Drohneneinheit ausgebildet.

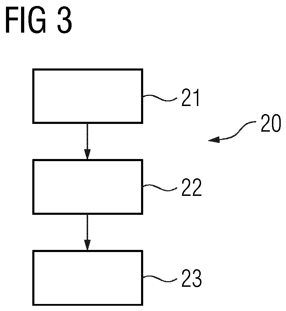

Die Fig. 3 zeigt eine beispielhafte Ausführung des eines erfindungsgemäßen Verfahrens 20. Das Verfahren 20 zur automatischen Einrichtung eines Radiologiearbeitsplatzes eines Bildgebungssystems 1 weist die Schritte des Lokalisierens 21, des mechanischen Aufnehmens 22 und des Positionierens 23 auf. Im Schritt des Lokalisierens 21 wird mindestens einer Komponente des Radiologiearbeitsplatzes lokalisiert. Im Schritt des mechanischen Aufnehmens 22 wird die Komponente mittels einer automatischen Manipulatoreinheit mechanisch aufgenommen. Im Schritt des Positionierens 23 wird die Komponente an einer für die Untersuchung vorgesehenen Position mittels der automatischen Manipulatoreinheit positioniert.

Obwohl die Erfindung im Detail durch das bevorzugte Ausführungsbeispiel näher illustriert wurde, so ist die Erfindung nicht durch die offenbarten Beispiele eingeschränkt und andere Variationen können vom Fachmann hieraus abgeleitet werden, ohne den Schutzumfang der Erfindung zu verlassen.

**Patentansprüche**

1. Manipulatorvorrichtung (2) zur automatischen Einrichtung eines Radiologiearbeitsplatzes eines Bildgebungssystems (1) aufweisend

- eine Sensoreinheit (5) zum Lokalisieren einer mindestens einen beweglichen Komponente (18) des Radiologiearbeitsplatzes, und
- eine automatische Manipulatoreinheit (3) zur mechanischen Aufnahme der Komponente und zur Positionierung der Komponente an einer für die Untersuchung vorgesehenen Position,
- wobei die Sensoreinheit ferner zur Komponentenerkennung ausgelegt ist,
- **dadurch gekennzeichnet, dass** die Manipulatorvorrichtung zum Datenaustausch mit dem Bildgebungssystem ausgebildet ist, um untersuchungsspezifische Informationen zu erhalten, um eine spezielle, zum Bildgebungssystem gehörige Komponente auszuwählen.

2. Manipulatorvorrichtung nach Anspruch 1, wobei die Komponente ein Röntgendetektor, ein Positionierungshilfsmittel, ein Raster oder eine Filtereinheit ist.

3. Manipulatorvorrichtung nach einem der vorangehenden Ansprüche, wobei die Sensoreinheit eine Kameraeinheit umfasst.

4. Manipulatorvorrichtung nach Anspruch 3, wobei die Sensoreinheit ferner zur Personenerkennung ausgebildet ist.

5. Manipulatorvorrichtung nach einem der vorangehenden Ansprüche, wobei die Sensoreinheit eine Positionselektronikeinheit umfasst.

6. Manipulatorvorrichtung nach einem der vorangehenden Ansprüche, wobei die Sensoreinheit ferner zur Kollisionsvermeidung ausgelegt ist.

7. Manipulatorvorrichtung nach einem der vorangehenden Ansprüche, wobei die Manipulatoreinheit eine vollautomatisch verfahrbare Untereinheit des Röntgensystems, eine Röntgenquellenbewegungseinheit (9) oder eine Röntgendetektorbewegungseinheit ist.

8. Manipulatorvorrichtung nach einem der Ansprüche 1 bis 6, wobei die Manipulatorvorrichtung eine Drohneneinheit ist.

9. Manipulatorvorrichtung nach einem der vorangehenden Ansprüche, wobei die Manipulatoreinheit eine Desinfektionseinheit umfasst.

10. Manipulatorvorrichtung nach einem der vorangehenden Ansprüche, wobei die Manipulatoreinheit eine Patientenhalteeinheit umfasst.

11. Manipulatorvorrichtung nach einem der vorangehenden Ansprüche, wobei mehrere Manipulatoreinheiten vorgesehen sind, welche kollaborativ miteinander agieren können.

12. Manipulatorvorrichtung nach einem der vorangehenden Ansprüche, ferner aufweisend eine Anzeigeeinheit zur Patientenpositionierung.

13. Medizinisches Bildgebungssystem (1) mit einer Manipulatorvorrichtung nach einem der vorangehenden Ansprüche.

14. Verfahren (20) zur automatischen Einrichtung eines Radiologiearbeitsplatzes eines Bildgebungssystems nach Anspruch 13 aufweisend die Schritte:

- Lokalisieren (21) mindestens einer Komponente des Radiologiearbeitsplatzes,
- mechanisches Aufnehmen (22) der Komponente mittels einer automatischen Manipulatoreinheit, und
- Positionieren (23) der Komponente an einer für die Untersuchung vorgesehenen Position mittels der automatischen Manipulatoreinheit.

**Claims**

1. Manipulator device (2) for automatic configuration of a radiology workstation of an imaging system (1) having

- a sensor unit (5) for localising an at least one moveable component (18) of the radiology workstation, and
- an automatic manipulator unit (3) for mechanical acquisition of the components and for positioning the components in a position intended for the examination,
- wherein the sensor unit is furthermore equipped for identifying components,
- **characterised in that** the manipulator device is designed for exchanging data with the imaging system in order to obtain items of information specific to the examination in order to select a particular component which is part of the imaging system.

2. Manipulator device according to claim 1, wherein the component is an X-ray detector, a positioning aid, a grid or a filter unit.

3. Manipulator device according to one of the preceding claims, wherein the sensor unit comprises a camera unit.

4. Manipulator device according to claim 3, wherein the sensor unit is furthermore designed to identify people.

5. Manipulator device according to one of the preceding claims, wherein the sensor unit comprises a positioning electronics unit.

6. Manipulation device according to one of the preceding claims, wherein the sensor unit is furthermore equipped to avoid collisions.

7. Manipulator device according to one of the preceding claims, wherein the manipulator unit is a fully automatic portable subunit of the X-ray system, an X-ray source motion unit (9) or an X-ray detector motion unit.

8. Manipulator device according to one of claims 1 to 6, wherein the manipulator device is a drone unit.

9. Manipulator device according to one of the preceding claims, wherein the manipulator unit comprises a disinfecting unit.

10. Manipulator device according to one of the preceding claims, wherein the manipulator unit comprises a patient holding unit.

11. Manipulator device according to one of the preceding claims, wherein a number of manipulator units are provided which can function with one another in a collaborative manner.

12. Manipulator device according to one of the preceding claims, further having a display unit for patient positioning.

13. Medical imaging system (1) with a manipulator device according to one of the preceding claims.

14. Method (20) for automatic configuration of a radiology workstation of an imaging system according to claim 13, having the steps:

- localising (21) at least one component of the radiology workstation,
- mechanically acquiring (22) the components by means of an automatic manipulator unit, and
- positioning (23) the components in a position intended for the examination by means of the automatic manipulator unit.

**Revendications**

1. Dispositif (2) manipulateur de mise en place automatique d'un poste de travail de radiologie d'un système (1) d'imagerie comportant

- une unité (5) de capteur pour la localisation d'au moins un composant (18) mobile du poste de travail de radiologie, et
- une unité (3) automatique de manipulateur pour la réception mécanique du composant et la mise en position du composant en une position prévue pour l'examen,
- dans lequel l'unité de capteur est conçue en outre pour l'identification du composant,

**caractérisé en ce que** le dispositif manipulateur est constitué pour l'échange de données avec le système d'imagerie afin d'obtenir des informations spécifiques à l'examen pour choisir un composant spécial appartenant au système d'imagerie.

2. Dispositif manipulateur suivant la revendication 1, dans lequel le composant est un détecteur de rayons X, un moyen auxiliaire de mise en position, une grille ou une unité de filtrage.

3. Dispositif manipulateur suivant l'une des revendications précédentes, dans lequel l'unité de capteur comprend une unité d'appareil photographique.

4. Dispositif manipulateur suivant la revendication 3, dans lequel l'unité de capteur est constituée en outre pour l'identification de personnes.

5. Dispositif manipulateur suivant l'une des revendications précédentes, dans lequel l'unité de capteur comprend une unité électronique de position.

6. Dispositif manipulateur suivant l'une des revendications précédentes, dans lequel l'unité de capteur est conçue en outre pour empêcher une collision.

7. Dispositif manipulateur suivant l'une des revendications précédentes, dans lequel l'unité de manipulateur est une sous-unité déplaçable entièrement automatiquement du système de rayons X, une unité (9) de déplacement d'une source de rayons X ou une unité de déplacement d'un détecteur de rayons X.

8. Dispositif manipulateur suivant l'une des revendications 1 à 6, dans lequel le dispositif manipulateur est une unité de drone.

9. Dispositif manipulateur suivant l'une des revendications précédentes, dans lequel l'unité de manipulateur comprend une unité de désinfection.

10. Dispositif manipulateur suivant l'une des revendications précédentes, dans lequel l'unité de manipulateur comprend une unité de maintien d'un patient.

11. Dispositif manipulateur suivant l'une des revendications précédentes, dans lequel il est prévu plusieurs unités de manipulateur, qui peuvent agir en collaboration les unes avec les autres.

12. Dispositif de manipulateur suivant l'une des revendications précédentes, comportant en outre une unité d'affichage pour la mise en position du patient.

13. Système (1) d'imagerie médicale comprenant un dispositif manipulateur suivant l'une des revendications précédentes.

14. Procédé (20) de mise en place automatique d'un poste de travail de radiologie d'un système d'imagerie suivant la revendication 13, comprenant les stades :

   - localisation (21) d'au moins un composant du poste de travail de radiologie,
   - réception (22) mécanique du composant au moyen d'une unité automatique de manipulateur, et
   - mise en position (23) au moyen de l'unité automatique de manipulateur du composant en une position prévue pour l'examen.

FIG 1

FIG 2

FIG 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102016225689 A1
- US 2016249991 A1
- JP H0271730 A
- DE 102014203465 A1
- EP 2302644 A2